# EUROPEAN PATENT SPECIFICATION

(11) **EP 1 377 180 B1**
(45) Date of publication and mention of the grant of the patent: **29.08.2007**
(21) Application number: 02761877.6
(22) Date of filing: 10.04.2002
(51) Int. Cl.: A23L 1/054, C12P 19/04, B01J 13/02

(54) **MICROCAPSULES**
MIKROKAPSELN
MICROCAPSULES

(30) Priority: 10.04.2001 DK 200100594
(43) Date of publication of application: 07.01.2004
(73) Proprietor: BASF AKTIENGESELLSCHAFT, 67056 Ludwigshafen (DE)
(72) Inventor: PEDERSEN, Anders, Vagn, DK-2830 Virum (DK); HANSEN, Morten, Mohr, DK-3450 Allerød (DK); MADSEN, Susanne, Lund, DK-2650 Hvidovre (DK); JENSEN, Nina, Musaeus, DK-2900 Hellerup (DK); HANSEN, Carsten, Lynggaard, DK-2730 Herlev (DK); KRISTENSEN STRARUP, Annette, DK-3460 Birkerød (DK); HANSEN, Karin, Meyer, DK-4681 Herfølge (DK)
(74) Representative: Olsen, Lars Pallisgaard
(86) International application number: PCT/DK2002/000238
(87) International publication number: WO 2002/082924

(56) References cited:
- EP-A1- 0 332 027
- EP-A2- 0 580 252
- WO-A1-00/17368
- WO-A1-00/58367
- WO-A1-00/70967
- WO-A1-95/02044
- WO-A2-98/28991
- US-A- 5 585 256
- US-A- 5 639 494

## Description

### FIELD OF THE INVENTION

The present invention relates to microcapsules comprising an active substance embedded in a novel type of matrix material, a process of preparing such microcapsules and products comprising the microcapsules.

### BACKGROUND OF THE INVENTION

Naturally occurring and modified polysaccharides and naturally occurring hydrocolloids such as alginate, carrageenan, gelatine, pectins, gum arabic and acacia gum, find wide spread use as matrix materials for the microencapsulation of sensitive active substances such as vitamins and aroma and flavour substances in food, food supplements, pharmaceuticals and agricultural products in order to protect them against influences of oxygen, moisture and irradiation as well as physical influences and thus to avoid chemical and/or physical degradation of said active substances and to improve their storage stability.

The hydrocolloids presently used as matrix materials are mainly of animal origin, e.g. gelatinous materials from mammals and fish.

However, microencapsulated products based on the use of matrix materials of animal origin are unacceptable for use in some products such as vegetarian foods, kosher foods and halal foods. Furthermore, the legislation governing the use of food products of animal origin is likely to become stricter in the future.

It has been attempted to use pectic substances as potential alternatives for matrix materials of animal origin, but it has been found that pectic substances such as citrus or sugar beet pectin only offer a limited protection of microencapsulated active substances against degradation and thus do not provide microencapsulated products with a desired storage stability.

European patent application No. 1 066 761 discloses encapsulated compositions comprising a fat-soluble substance encapsulated in a carbohydrate matrix composed of maltose or maltose syrup or a mixture of low-molecular weight carbohydrates, such as maltodextrin, optionally in combination with a high-molecular weight carbohydrate, an emulsifier and optionally an antioxidant.

US patent No. 5 998 176 discloses a method of causing gelling or increase of viscosity of an aqueous medium containing a pectic material such as sugar beet pectin by treating the aqueous medium with a carboxylic ester hydrolase and an oxidase and/or a peroxidase in the presence of an oxidizing agent for use with said oxidase and/or peroxidase. It is mentioned that the resulting gelled or viscous products are suitable, e.g. as thickening and/or stabilizing agents in foodstuff applications, as a material for drug encapsulation in medical/medicinal applications and as a slow release vehicle in both medical/medicinal and agricultural/horticultural applications.

Danish Patent Application 1991 01060 discloses unbranched arabans, e.g. from sugar beet, obtained by treatment of sugar beet araban with α-L-arabinofuranosidase, and their use as gelling agents, as emulsifiers and as encapsulating material. The sugar beet arabans used as enzyme substrate in that process are obtained by an alkaline treatment of sugar beet pulp, and the raw sugar beet arabans comprises typical about 70-85 % arabinose, 5-10 % uronic acid, 8-15 % D-galactose, and a few % rhamnose and other monosaccharides.

WO 00/70967 A1 discloses a composition comprising colouring substance bodies that are at least partially coated with unmodified pectin selected from beet pectin, chicory pectin and Jerusalem artichoke, preferable with a high degree of acetylation.

WO 00/17368 discloses an orange fruit pectin acetyl esterase and a process wherein the esterase is contacted with a substrate, such as pectin from a fruit or a vegetable. Improved gelling properties are obtained by deacetylating sugar beet pectin with the disclosed acetyl esterase.

The object of the present invention is to provide microcapsules comprising a matrix material of non-animal origin and being capable of effectively protecting active substances embedded in the matrix material against chemical and physical influences.

### SUMMARY OF THE INVENTION

The microcapsules according to the invention are characterized in that the matrix material is obtainable by treating a pectic substance with one or more enzymes selected from the group consisting of esterases (E.C.3.1), glucosidases (E.C.3.2), peptidases (E.C.3.4), proteases (E.C.3.4) and lyases (E.C.4).

The improved stability properties obtained with microcapsules of the invention compared to microcapsules comprising a matrix material of non-modified pectin will appear from the drawings in which
- Fig. 1: shows stability test results obtained by storage of microcapsules at a temperature of 25 °C and 60 % RH, and
- Fig. 2: shows stability test results obtained by storage of microcapsules at a temperature of 40 °C and 75 % RH.

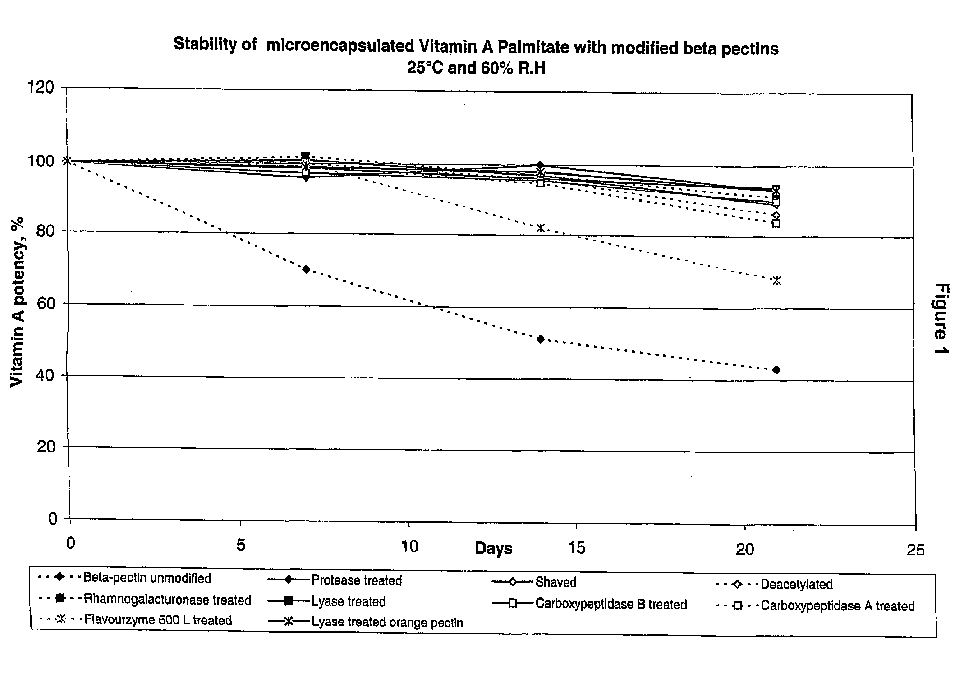

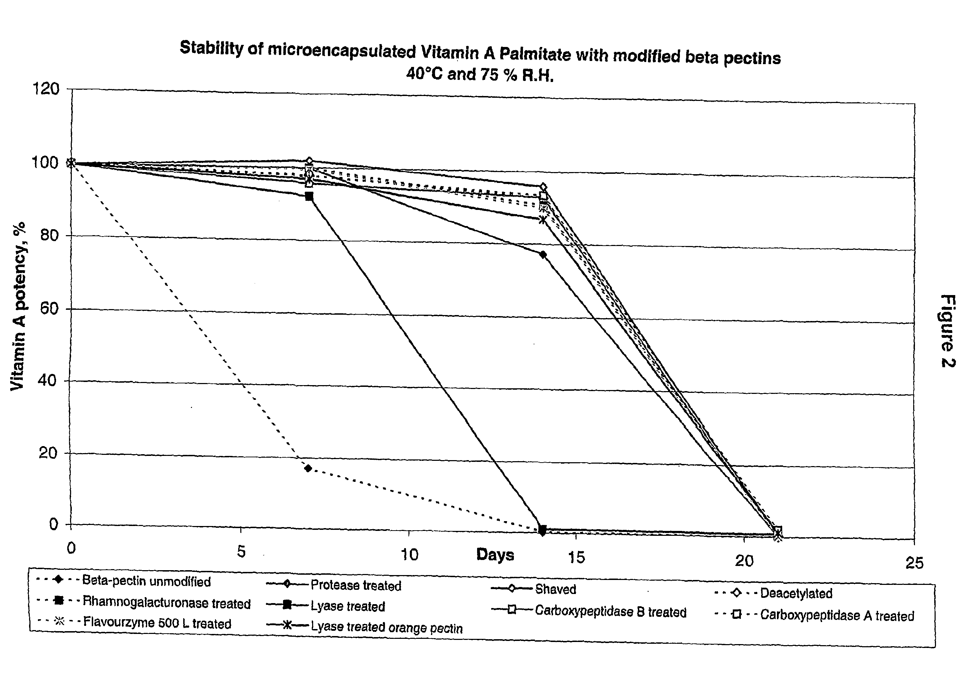

Figs. 1 and 2 show curves illustrating the potency of microcapsules containing vitamin A palmitate embedded in various matrix materials as a function of time.

It appears from said curves that microcapsules according to the invention, viz. comprising matrix material being protease treated pectin, shaved β-pectin, deacetylated β-pectin, rhamnogalacturonase treated β-pectin, lyase treated β-pectin, carboxypeptidase A or B treated β-pectin, and Flavourzyme 500L^{™} treated β-pectin as well as lyase treated orange pectin exhibit significantly better stabilities when stored both at 25 °C, 60% RH and at 40 °C, 75% RH for periods up to at least 20 days than microcapsules comprising non-modified pectin as matrix material.

The term "microcapsules" as used herein means particles each comprising a matrix material having embedded therein a plurality of solid or liquid micro particles.

Pectins are high-molecular weight polygalacturonic acids joined by (1⇒4)-α-glycosidic links in which some of the carboxylic acid groups are esterified with methanol and they are composed of flexible regions in which the polymer backbone is rich in rhamnose and have side chains, "hairy regions", of a complex nature.

Pectins also comprise "smooth regions" which are rigid regions in which the backbone consists essentially of galacturonic acid or galacturonic acid residues having very small side chains such as methyl or ethyl groups.

US Patent No. 5 929 051 contains a more detailed discussion of the general structure of pectin and pectic substances as presently understood.

As used in connection with the present application the term "pectic substance" encompasses pectin, pectic acid and salts and esters of pectic acid (pectates), whereby the pectic substance has a galacturonic acid content of above 40 %.

The galacturonic acid content of the pectic substance is preferable above 50 % and more preferred above 65 %.

The matrix material of the microcapsules of the invention preferably consists of pectin, which has been treated with one or more enzymes capable of modifying of the hairy regions, viz. the side chains of the rhamnogalacturonan backbone of the pectin.

Preferred examples of such enzymes are rhamnogalacturonase, rhamnogalacturonan acetyl esterase, β-galactosidase, arabinanase, galactanase and α-arabinofuranosidase.

Another preferred matrix material is a pectin which has been treated with one or more enzymes capable of modifying the backbone of the smooth regions of pectin so as to form separate elements comprising hairy regions.

Examples of such enzymes are pectin lyase and combinations of polygalacturonase and pectin methyl esterase.

Most pectins contain proteins, e.g. in an amount of about 1-5% w/w and surprisingly it has been found that the protective properties of such protein-containing pectins are significantly improved, if they are treated with a protease such as papain, pepsin and trypsin.

The pectic material to be modified according to the invention is preferably derived from beetroot (Beta vulgaris L. Chenopodiaceae), including sugar beet, garden beet (red beet), chard, mangel, spinach beet, silver beet and fodder beet, or from orange, grape, soybean, linseed, Jerusalem artichoke, celery, and potatoes. Sugar beet pectin is a particularly useful pectic substance.

The enzyme classification system used in the definition of enzymes for use for modifying pectin substances according to the present invention is described in Enzyme Nomenclature 1992, Academic Press, San Diego, California, with supplements.

Esterases (E.C.3.1) constituting a subclass to hydrolases (E.C.3) are enzymes that catalyse the hydrolysis of ester groups of pectin. Preferred esterases for use for the modification of pectic substances are deacetylating enzymes, such as rhamnogalacturonan-acetylesterase, pectin acetyl esterase, and pectin methyl esterase.

Glucosidases (E.C.3.2), also constituting a subclass to hydrolases (E.C.3), are enzymes, which catalyse the hydrolysis of glycosidic bonds in pectin. Preferred glucosidases are debranching enzymes, such as α-arabinofuranosidase, galactanase, arabinanase, and endo- and exopolygalacturonases. A mixture of α-arabinofuranosidase, galactanase, rhamnogalacturonase, and arabinanase is particularly useful.

Peptidases and proteases (E.C.3.4), which also constitute a subclass to hydrolases, (E.C.3) catalyse the hydrolysis of peptide bonds. Preferred proteases are papain, pepsin and trypsin (endopeptidases), and carboxypeptidase A and B (exopeptidases), as well as combinations of endo- and exopeptidases, such as Flavorzyme 500L^{™}.

Examples of suitable commercial proteases are Papain 16000 from Valley Research and Collupulin® from DSM Gist-Brocades Food Specialities.

Lyases (E.C.4) are enzymes, which catalyse addition to double bonds. A preferred lyase is pectinase PL lyase.

The term "deacetylating enzyme" means an enzyme that is capable of removing acetyl groups, which are covalently bonded to galacturonic acid residues in the backbone of the hairy regions of pectin.

The terms "shaving enzyme" or "debranching enzyme" mean enzymes which are capable of reducing the length of the side chains of the hairy regions of pectin.

The term "pectin esterase" means an enzyme, which is capable of removing ester residues from galacturonic acid residues in the backbone of pectin.

In practice the enzymatic modification of pectic substances may be carried out as follows:
Temperature and pH of the pectin preparation are adjusted to working temperature and pH of the enzyme to be used, respectively. Enzyme is dissolved/diluted in ion exchanged water and added to the pectin preparation. Reaction is carried out while stirring continuously, and if necessary pH is controlled by titration. After a certain time reaction is terminated by lowering pH. In order to irreversibly inactivate the enzyme, temperature is raised to 80°C for 10 min. Temperature of the solution is lowered and the pectin is precipitated (1:3) in 80% 2-propanol. The precipitated pectin is drained on a belt press and put in a drying cabinet at 70°C for 24 hours. After drying the pectin is ground and sieved (DIN 24).

The pectin preparation to be used as substrate for enzyme treatment can be an extract directly obtained from the raw material e.g. sugar beet peel or it can be a solution of a refined pectin product.

An extract of sugar beet pectin may be prepared as follows:
1) Mixing dry granular beet pulp with an aqueous solution of a strong, mineral acid, preferably nitric acid
2) Extracting the pulp with rigorous agitation for about one to five hours at 60-80 °C and pH ranging from 1.5 to 2.5.
3) Separating the resulting mixture into waste solids and a liquid containing pectin
4) Treating the liquid containing pectin with enzyme as described above.

A pectin solution is made by adding pectin powder to hot (70°C) ion-exchanged water. The preparation is stirred continuously, until the pectin is completely dissolved.

The active substances contained in the microcapsules of the present invention may be any substance, which during storage, transport, handling and use requires protection, e.g. from oxygen, moisture, light radiation, and physical influences, in order to avoid physical and chemical decomposition of the substance. These active substances are further defined as being active in either a chemical or biological system. Furthermore, a protective matrix may be used to prevent the active substance from reacting with other substances present in a composition or with substances with which it may come into contact during use and which would have a deleterious affect upon the active substance's desired activity. Also, a protective matrix may be used to transform liquids and other substances, which are difficult to handle, e.g. due to stickiness, into a solid form suitable for handling and processing during use, such as a powder of microcapsules.

Examples of active substances suitable for use in the present invention are fat-soluble substances, such as vitamins, fatty acids, e.g. mono- and polyunsaturated fatty acids, which may be added in the form of fish oil containing i.a. the (n-3) fatty acids docosahexaenoic acid (DHA) and eicosapentaenoic acid (EPA), and in the form of evening primrose oil and castor oil containing i.a. the (n-6) fatty acid γ-linolenic acid, carotenoides, e.g. β-caroten, lutein, lycopene, β-cryptoxanthin and zeaxanthin, oils and fats; water-soluble substances, such as vitamin C; enzymes, e.g. amylase; pharmaceuticals, such as griseofulvin, ibuprofen, benzodiazepines, phenacetin, hormones and paracetamol; and other nutritional supplements, such as minerals.

Additional active substances are aroma and flavour compounds.

The matrix material of the microcapsules of the invention may contain conventional additives such as antioxidants, e.g. t-butylhydroxyloluene (BHT), t-butylhydroxyanisole (BHA), ascorbic acid, ascorbyl palmitate, sodium ascorbate, tocopherols, TBHQ, ethoxyquine, propyl gallate, and extracts from herbs, i.a. rosemary extract; powdering agents, e.g. starches, modified starches, tri-calcium phosphate, lactose, mannitol, ethylcellulose, coagulated albumin, hardened gelatine, casein, stearat-Ca, stearat-Na, metal soaps, hydrogenated ricinus oil, polyoxide, talcum, waxes, and silicates; anti-caking agents, e.g. tri-calcium phosphate and silicates, i.a. silicon dioxide and sodium aluminium silicate; plasticizers, e.g. carbohydrates and carbohydrate alcohols, examples of which are saccharose, glucose, fructose, lactose, invert sugar, sorbitol, mannitol, maltodextrin, glycerin, and mixtures thereof, preferably saccharose, lactose, maltodextrin, and mixtures thereof.

The invention also relates to a process of preparing microcapsules containing an active substance embedded in a matrix which process comprises the steps of providing an aqueous medium of a pectic substance modified by treatment with one or more enzymes selected from the group consisting of esterases (E.C.3.1), glucosidases (E.C.3.2), peptidases (E.C.3.4), proteases (E.C.3.4), and lyases (E.C.4), adding to said solution at least one active substance, finely dividing and drying the mixture thus obtained to obtain a mass of particles each containing a plurality of liquid or solid micro particles of the active substance embedded in a matrix comprising a modified pectic substance.

The final step of the above process may be carried out with conventional methods such as spray cooling, spray drying, modified spray drying or sheet drying and crushing, cf. WO 91/06292.

The present invention also relates to products comprising the microcapsules described above. Typical examples of such products are foods, food supplements, beverages, pharmaceutical and veterinary products, feeds, feed supplements, personal care products and household products.

The invention will now be described in further detail with reference to the following examples.

### EXAMPLES

In the examples the molecular weight (MW) is measured by the Capillary Tube Method principle as follows:
The outlet time is measured for a pectin/hexametaphosphate solution and the molecular weight is thereafter calculated after a well-known formula (see WO 00/58367 Pectin having reduced calcium sensitivity, page 12).

The outlet time is measured on two outlets. If the difference between the times is more than 0.4 seconds the measuring is repeated until the difference is less than the appropriate value. The outlet time used for the molecular determination is the mean value of the above-mentioned identical or substantially identical measuring results.

### Preparation of enzymatically modified pectic substances

### Example 1

In this example the pectic substance was derived from sugar beets (GENU beta pectin, lot 92455, produced by CP Kelco ApS, Lille Skensved, Denmark) and the enzyme was papain (Collupulin® papain batch R9741, produced by DSM Gist-Brocades Food Specialities, Delft, The Netherlands).

1000 l of ion-exchanged water was heated to 70 °C, 0.4 M of NaCl was dissolved and 20 kg of pectic substance was added, while stirring continuously. After the pectin was completely dissolved, temperature was lowered to 45°C; pH was adjusted to 5.50 by titration with a 2% (w/v) NH₃ solution. 240 grams of Collupulin was dissolved in approximately 5 l ion exchanged water at ambient temperature and added to the pectin solution. During the experiment pH was kept constant at 5.50 by titration with 2% (w/v) NH₃. After 20 minutes, 6127 ml of 2% (w/v) NH₃ was added, and the reaction was stopped by addition of a 10% HNO₃ solution till pH 2.50. In order to irreversibly inactivate the enzyme, the temperature was raised to 80 °C. After 10 minutes at 80 °C, the solution was cooled to 50 °C and the modified pectin was precipitated (1:3) in 80% 2-propanol. The precipitated pectin was drained on a belt press and put into a drying cabinet at 70 °C for 24 hours.

After drying the pectin was ground and sieved (DIN 24). Degree of acetylation (%D(Ac)), degree of esterification (%DE), galacturonic acid content (%GA) and molecular weight (MW) of the enzyme treated pectin were determined and the results obtained will appear from Table 1.

**TABLE 1**

| | Collupulin treated sugar beet pectin |
|---|---|
| %D(Ac) | 19.9 |
| %DE | 38.8 |
| %GA | 76.9 |
| MW | 63000 Da |

### Example 2

In this example the pectic substance was derived from sugar beets (GENU® beta pectin, lot 82899, produced by CP Kelco ApS, Lille Skensved, Denmark). The enzymes used were α-arabinofuranosidase (α-ARA) (batch sp 580, PPJ 4494), arabinanase (batch sp 564, PPJ 4381) and galactanase (batch sp 518 PPJ 4368), all produced by Novo Nordisk, Bagsvaerd, Denmark.

1000 l of ion-exchanged water was heated to 60°C and 10 kg of pectic substance was added, while stirring continuously. After the pectin was completely dissolved, the temperature was lowered to 45 °C, and pH was adjusted to 4.50 by titration with a 2% (w/v) NH₃ solution. 5 grams of arabinanase, 35 grams of α-arabinofuranosidase and 35 grams of galactanase was diluted in approximately 1l ion exchanged water at ambient temperature and added to the pectin solution. After 4 hours, reaction was stopped by addition of a 10% (w/v) HNO₃ solution till pH 3.00. In order to irreversibly inactivate the enzyme, the temperature was raised to 80 °C. After 10 minutes at 80 °C, the solution was cooled to 20 °C, and the modified pectin was precipitated (1:3) in 80% 2-propanol. The precipitated pectin was drained on a belt press and put into a drying cabinet at 70 °C for 24 hours. After drying the pectin was ground and sieved (DIN 24). Degree of acetylation (%D(Ac)), degree of esterification (%DE), galacturonic acid content (%GA), molecular weight (MW) and neutral sugar content of the enzyme treated pectin sample were determined and the results obtained will appear from Table 2.

**TABLE 2**

| Sample | α-ARA, arabinanase, and galactanase treated sugar beet pectin |
|---|---|
| %DE | 60 |
| %GA | 81 |
| MW | 66000 Da |
| %Rhamnose | 7 |
| %Arabinose | 2 |
| %Galactose | 8 |
| %Other sugars | 2 |

### Example 3

In this example the pectic substance was derived from sugar beets (GENU® beta pectin, lot 92455 produced by CP Kelco ApS, Lille Skensved, Denmark) and the enzyme was rhamnogalacturonan acetyl esterase (batch PPJ 4456, produced by Novo Nordisk, Bagsvaerd, Denmark).

50 l of ion-exchanged water was heated to 70 °C, and 1 kg of pectic substance was added, while stirring continuously. The temperature was lowered to 50 °C, and pH was adjusted to 4.50 by titration with a 2% (w/v) NH₃ solution. 10 grams of rhamnogalacturonan acetyl esterase was added to the pectin solution. After 24 hours at 50 °C, while stirring continuously, the reaction was stopped by addition of a 10% (w/v) HNO₃ solution till pH 2.50. In order to irreversibly inactivate the enzyme, the temperature was raised to 80 °C. After 10 minutes at 80 °C, the solution was cooled to 50 °C, and the modified pectin was precipitated (1:3) in 80% 2-propanol. The precipitated pectin was drained on a belt press and put into a drying cabinet at 70 °C for 24 hours. After drying the pectin was ground and sieved (DIN 24). Degree of acetylation (%D(Ac)), degree of esterification (%DE), galacturonic acid content (%GA) and molecular weight (MW) were determined and the results obtained will appear from Table 3.

**TABLE 3**

| Sample | Rhamnogalacturonan acetylesterase treated sugar beet pectin |
|---|---|
| %D(Ac) | 15.2 |
| %DE | 52.7 |
| %GA | 72.5 |
| MW | 57500 Da |

### Example 4

In this example the pectic substance was derived from sugar beets (GENU® beta pectin, lot 82899 produced by CPKelco ApS, Lille Skensved, Denmark) and the enzyme was rhamnogalacturonase (Batch PPJ 4478, produced by Novo Nordisk, Bagsvaerd, Denmark).

50 l of ion-exchanged water was heated to 70 °C, and 1 kg of pectic substance was added, while stirring continuously. The temperature was lowered to 50 °C, and pH was adjusted to 4.50 by titration with a 2% (w/v) NH₃ solution. 3.125 grams of rhamnogalacturonase was dissolved in approximately 50 ml of ion-exchanged water and added to the pectin solution. After 4 hours at 50 °C, the reaction was stopped by addition of a 10% (w/v) HNO₃ solution till pH 2.50. In order to irreversibly inactivate the enzyme, the temperature was raised to 80 °C. After 10 minutes at 80 °C, the solution was cooled to 50 °C, and the modified pectin was precipitated (1:3) in 80% 2-propanol. The precipitated pectin was drained on a belt press and put into a drying cabinet at 70 °C for 24 hours. After drying the pectin was ground, and finally the pectin was sieved (DIN 24). Degree of acetylation (%D(Ac)), degree of esterification (%DE), galacturonic acid content (%GA) and molecular weight (MW) were determined and the results obtained will appear from Table 4.

**TABLE 4**

| Sample | Rhamnogalacturonase treated sugar beet pectin |
|---|---|
| %D(Ac) | 16.5 |
| %DE | 58.7 |
| %GA | 81.1 |
| MW | 64000 Da |

### Example 5

In this example the pectic substance was derived from sugar beet (Batch no. 30003, Type SF H-25, produced by CPKelco, Germany GmbH, Grossenbrode, Germany) and the enzyme was Enzeco Pectinase PL lyase from Enzyme Development Corporation, Batch no. S-11677, Activity 26 U/ml.

55 l of ion-exchanged water was heated to 70 ° C and 2,75 kg of pectic substance was added, while stirring continuously. After the pectin was completely dissolved, the temperature was lowered to 45 ° C; pH was adjusted to 4.50 by titration with a 10% (^{w}/v) soda solution. 1,63 ml Enzeco Pectinase PL lyase was added to the pectin solution. During the experiment the pH was kept constant at 5,5 by titration with 5% (^{w}/v) soda. After 6 hours (viscosity constant, 9 cP) the reaction was stopped by addition of a 10% (^{w}/v) soda solution till pH 2,50. In order to irreversibly inactivate the enzyme, the temperature was raised to 80 °C. After 10 minutes at 80 ° C, the solution was cooled to 50 °C, evaporated to half amount by the following procedure: The solution was transferred to the evaporator. Heat was applied under vacuum at 0.8 bar, and the solution will reach the boiling point (around 60 ° C). The solution was cooled to 50 ° C and the modified pectin was precipitated (1:3) in 80% 2-propanol. The precipitated pectin was drained on a belt press and put into a drying cabinet at 70 ° C for 24 hours. After drying, the pectin was ground and sieved (DIN 24). Degree of acetylation (% D(Ac)), degree of esterification (% DE). Galacturonic acid (%GA) and molecular weight (MW) of the enzyme treated pectin were determined and the result appears in Table 5:

**TABLE 5**

| Sample | Pectinase PL lyase treated sugar beet pectin |
|---|---|
| % DAc | 22,5 |
| % DE | 51,7 |
| % GA | 70,0 |
| MW | 17.900 Da |

### Example 6

In this example the pectic substance was derived from sugar beets (GENU Beta pectin, lot 30003, produced by CP Kelco, Germany GmbH, Grossenbrode, Germany) and the enzyme was Carboxypeptidase B from Sigma, Batch no. 108H7406, activity 176u/mg

40 l of ion-exchanged water was heated to 70 ° C and 1,6 kg of pectic substance was added, while stirring continuously. After the pectin was completely dissolved, the temperature was lowered to 45 ° C; pH was adjusted to 7.50 by titration with a 10% (^{w}/v) soda solution. 21 mg of Carboxypeptidase B was added to the pectin solution. During the experiment the pH was kept constant at 7,5 by titration with 5% (^{w}/v) soda. After 24 hours the reaction was stopped by addition of a 10% (^{w}/v) soda solution till pH 2,50. In order to irreversibly inactivate the enzyme, the temperature was raised to 80 °C. After 10 minutes at 80 °C, the solution was cooled to 50 °C and the modified pectin was precipitated (1:3) in 80% 2-propanol. The precipitated pectin was drained on a belt press and put into a drying cabinet at 70 ° C for 24 hours. After drying, the pectin was ground and sieved (DIN 24). Degree of acetylation (% D(Ac)), degree of esterification (% DE). Galacturonic acid (%GA) and molecular weight (MW) of the enzyme treated pectin were determined and the result appears in Table 6.

**TABLE 6**

| Sample | Carboxypeptidase B treated sugar beet pectin |
|---|---|
| % DAc | 24,6 |
| % DE | 51,8 |
| % GA | 72,9 |
| MW | 69.100 Da |

### Example 7

In this example the pectic substance was derived form sugar beets (GENU Beta pectin, lot 30003, produced by CPKelco, Germany GmbH, Grossenbrode, Germany) and the enzyme was Carboxypeptidase A from Sigma, Batch no. 127H7445, activity 50u/mg.

40 l of ion exchanged water was heated to 70 ° C and 1,6 kg of pectin was added, while stirring continuously. After the pectin was completely dissolved, the temperature was lowered to 45 ° C; pH was adjusted to 7.50 by titration with a 10% (^{w}lv) soda solution. 21 mg of Carboxypeptidase A was added to the pectin solution. During the experiment the pH was kept constant at 7,5 by titration with 5% (^{w}/v) soda. After 24 hours the reaction was stopped by addition of a 10% (^{w}/v) soda solution till pH 2,50. In order to irreversibly inactivate the enzyme, the temperature was raised to 80 ° C. After 10 minutes at 80 ° C, the solution was cooled to 50 ° C and the modified pectin was precipitated (1:3) in 80% 2-propanol. The precipitated pectin was drained on a belt press and put into a drying cabinet at 70 °C for 24 hours. After drying, the pectin was ground and sieved (DIN 24). Degree of acetylation (% D(Ac)), degree of esterification (% DE). Galacturonic acid (%GA) and molecular weight (MW) of the enzyme treated pectin were determined and the result appears in table 7.

**TABLE 7**

| Sample | Carboxypeptidase A treated sugar beet pectin |
|---|---|
| % DAc | 17,8 |
| % DE | 37,5 |
| % GA | 74,6 |
| MW | 14.100 |

### Example 8

In this example the pectic substance was derived from sugar beets (GENU Beta pectin, lot 30003, produced by CPKelco, Germany GmbH, Grossenbrode, Germany) and the enzyme was Flavorzyme 500L^{™} from Novozymes, Denmark, Batch no. HPN01200 with an activity of 500 LAPU/g.

50 l of ion-exchanged water was heated to 70 ° C, and 2 kg of pectic substance was added, while stirring continuously. After the pectin was completely dissolved, the temperature was lowered to 45 ° C; pH was adjusted to 5.50 by titration with a 10% (^{w}/v) soda solution. 15 ml of Flavorzyme 500L was added to the pectin solution. During the experiment the pH was kept constant at 5,5 by titration with 5% (^{w}/v) soda. After 4 hours the reaction was stopped by addition of a 10% (^{w}/v) soda solution till pH 2,50. In order to irreversibly inactivate the enzyme, the temperature was raised to 80 ° C. After 10 minutes at 80 ° C, the solution was cooled to 50 ° C and the modified pectin was precipitated (1:3) in 80% 2-propanol. The precipitated pectin was drained on a belt press and put into a drying cabinet at 70 ° C for 24 hours. After drying, the pectin was ground and sieved (DIN 24). Degree of acetylation (% D(Ac)), degree of esterification (% DE). Galacturonic acid (%GA) and molecular weight (MW, capillary tune method) of the enzyme treated pectin were determined and the result appears in Table 8.

**TABLE 8**

| Sample | Flavorzyme treated sugar beet pectin |
|---|---|
| % DAc | 22.8 |
| % DE | 55.0 |
| % GA | 73.8 |
| MW | 61.000 Da |

### Example 9

In this example the pectic substance was derived from orange (Batch no. 1001-69-1, produced by CPKelco, Limeira, Brasil) and the enzyme was Enzeco Pectinase PL lyase from Enzyme Development Corporation, Batch no. S-11677, Activity 26 U/ml.

55 l of ion-exchanged water was heated to 70 ° C and 940 gram pectic substance was added, while stirring continuously. After the pectin was completely dissolved, the temperature was lowered to 45 ° C; pH was adjusted to 4.50 by titration with a 10% soda solution. 0,55 ml Enzeco Pectinase PL lyase was added to the pectin solution. During the experiment the pH was kept constant at 5,5 by titration with 5% (^{w}/v) soda. After 1,5 hours the reaction was stopped by addition of a 10% (^{w}/v) soda solution till pH 2,50. In order to irreversibly inactivate the enzyme, the temperature was raised to 80 ° C. After 10 minutes at 80 ° C, the solution was cooled to 50 ° C, evaporated to half amount by the following procedure: The solution was transferred to the evaporator. Heat was applied under vacuum at 0.8 bar, and the solution will reach the boiling point (around 60 °C). The solution was cooled to 50 ° C and the modified pectin was precipitated (1:3) in 80% 2-propanol. The precipitated pectin was drained on a belt press and put into a drying cabinet at 70 ° C for 24 hours. After drying, the pectin was ground and sieved (DIN 24). Degree of acetylation (% D(Ac)), degree of esterification (% DE). Galacturonic acid (%GA) and molecular weight (MW) of the enzyme treated pectin were determined and the result appears in table 9:

**TABLE 9**

| Sample | Pectinase PL lyase treated orange pectin |
|---|---|
| % DAc | 2.4 |
| % DE | 68.9 |
| % GA | 87.5 |
| MW | 25.400 |

### Preparation of microcapsules

The microcapsules were prepared in accordance with the following recipe:
1000.00 grams water
92.9 grams (modified) pectin
797.0 grams sugar
12.6 grams sodium ascorbate
400.0 grams Vitamin A palmitate oil
20.4 grams α-tocopherol

### Comparative Example

92.9 grams of sugar beat pectin (GENU® beta pectin type BETA, lot HF 72-097-0, from CP Kelco ApS, Lille Skensved, Denmark) and 797.0 grams of saccharose were dissolved in 1.0 l water at 65°C in an emulsion tank and 12.6 grams of sodium ascorbate added. A mixture of 400.0 gram Vitamin A palmitate 1.7 million lU/g and 20.4 gram DL-α-tocopherol was heated to 65 °C in a beaker. The oily mixture was added to the aqueous solution of pectin, sugar and sodium ascorbate under slow agitation and then stirred vigorously at 10.000 rpm by Ultra Turrax (Ultra Turrax T50) for 60 minutes at 65 °C. The final emulsion was diluted with 975 ml 65 °C water to a viscosity of 150 cP (measured by Brookfield Viscometer type HAT, spindle HA1). The mean oil droplet size was measured to be 1.40 µm (Malvern Mastersizer Long bed ver. 2.19, focal length 45 mm, beam length 2.4 mm). Subsequently the emulsion was atomized in a spray tower, where the droplets were covered with starch and dried. Only part of the emulsion was sprayed. This yielded after screening on mesh 30/120 approx. 1 kg of particulate product with a potency of 105.000 lU/g (The sample was saponified with 50% KOH, 96% ethanol and a 10% sodium ascorbate solution and extracted with heptane. The amount of Vitamin A was measured by HPLC, Hichrom LiChrosorb CN-5, 5 µl, 250 mm x 4.0 mm against an external standard).

The stability of the product was investigated as follows: Approximately 0.2 gram product was weighed and placed in a small open glass container (15 x 10 mm) and kept at 25°C/60% R.H. and at 40°C/75% R.H. for 3 weeks. Samples were analyzed at start, after 7.14 and 21 days for potency. The results obtained are given below:

| Potency: | 25°C/60%R.H. | 40°C/75%R.H. |
|---|---|---|
| Start: | 100% | 100% |
| 7 days: | 70% | 17% |
| 14 days: | 51 % | 0.3% |
| 21 days: | 43% | 0% |

### Example 10

92.9 grams of Collupulin modified sugar beet pectin prepared as described in Example 1 and 797.0 grams of saccharose were dissolved in 1.0 l water at 65 °C in an emulsion tank and 12.6 grams of sodium ascorbate added. A mixture of 400.0 grams Vitamin A palmitate 1.7 million IU/g and 20.4 grams DL-α-tocopherol was heated to 65 °C in a beaker. The oily mixture was added to the aqueous solution of modified pectin, sugar and sodium ascorbate under slow agitation and then stirred vigorously at 10.000 rpm by Ultra Turrax (Ultra Turrax T50) for 60 minutes at 65 °C. The final emulsion was diluted with 1000 ml 65 °C water to a viscosity of 155 cP (measured by Brookfield Viscometer type HAT, spindle HA1). The mean oil droplet size was measured to be 1.09 µm (Malvern Mastersizer Long bed ver. 2.19, focal length 45 mm, beam length 2.4 mm). Subsequently the emulsion was atomised in a spray tower, where the droplets were covered with starch and dried. Only part of the emulsion was sprayed. This yielded after screening on mesh 30/120 approx. 1 kg of particulate product with a potency of 278.000 lU/g (The sample was saponified with 50% KOH, 96% ethanol and a 10% sodium ascorbate solution and extracted with heptane. The amount of Vitamin A was measured by HPLC, Hichrom LiChrosorb CN-5, 5 µl, 250 mm x 4.0 mm against an external standard).

The stability of the product was investigated as follows: Approximately 0.2 gram product was weighed and placed in a small open glass container (15 x 10 mm) and kept at 25°C/60% R.H. and at 40°C/75% R.H. for 3 weeks. Samples were analyzed at start, after 7, 14 and 21 days for potency. The results obtained are given below:

| Potency: | 25°C/60% R.H. | 40°C/75% R.H. |
|---|---|---|
| Start: | 100% | 100% |
| 7 days: | 96% | 100% |
| 14 days: | 100% | 77% |
| 21 days: | 93% | 0.1% |

As will appear from these results the potency and the stability of the composition of the invention were far superior to those of the prior art composition of the Comparative Example.

### Example 11

92.9 grams of α-ARA, arabinanase, and galactanase modified sugar beet pectin prepared as described in Example 2 and 797.0 grams of saccharose were dissolved in 1.0 l water at 65 °C in an emulsion tank and 12.6 grams of sodium ascorbate added. A mixture of 400.0 grams Vitamin A palmitate 1,7 million lU/g and 20.4 grams DL-α-tocopherol was heated to 65 °C in a beaker. The oily mixture was added to the aqueous solution of modified pectin, sugar and sodium ascorbate under slow agitation and then stirred vigorously at 10,000 rpm by Ultra Turrax (Ultra Turrax T50) for 60 minutes at 65°C. The final emulsion was diluted with 1050 ml 65 °C water to a viscosity of 155 cP (measured by Brookfield Viscometer type HAT, spindle HA1). The mean droplet size was measured to be 1.28 µm (Malvern Mastersizer Long bed ver. 2.19, focal length 45 mm, beam length 2.4 mm). Subsequently the emulsion was atomized in a spray tower, where the droplets were covered with starch and dried. Only part of the emulsion was sprayed. This yielded after screening on mesh 30/120 approx. 1 kg of particulate product with a potency of 224,000 lU/g (The sample was saponified with 50% KOH, 96% ethanol and a 10% sodium ascorbate solution and extracted with heptane. The amount of Vitamin A was measured by HPLC, Hichrom LiChrosorb CN-5, 5 µl, 250 mm x 4.0 mm against an external standard).

The stability of the product was investigated as follows: Approximately 0.2 gram product was weighed and placed in a small open glass container (15 x 10 mm) and kept at 25°C/60% R.H. and at 40°C/75% R.H. for 3 weeks. Samples were analyzed at start, after 7, 14 and 21 days for potency. The results obtained are given below:

| Potency: | 25°C/60% R.H. | 40°C/75% R.H. |
|---|---|---|
| Start: | 100% | 100% |
| 7 days: | 101% | 102% |
| 14 days: | 97% | 96% |
| 21 days: | 89% | 0.4% |

As will appear from these results the potency and the stability of the composition of the invention were far superior to those of the prior art composition of the Comparative Example.

### Example 12

92.9 grams of rhamnogalacturonan acetylesterase modified sugar beet pectin prepared as described in Example 3 and 797.0 grams of saccharose were dissolved in 1.0 l water at 65°C in an emulsion tank and 12.6 grams of sodium ascorbate added. A mixture of 400.0 grams Vitamin A palmitate 1,7 million IU/g and 20.4 grams DL-α-tocopherol was heated to 65°C in a beaker. The oily mixture was added to the aqueous solution of modified pectin, sugar and sodium ascorbate under slow agitation and then stirred vigorously at 10,000 rpm by Ultra Turrax (Ultra Turrax T50) for 60 minutes at 65 °C. The final emulsion was diluted with 800 ml 65 °C water to a viscosity of 150 cP (measured by Brookfield Viscometer type HAT, spindle HA1). The mean oil droplet size was measured to be 1.59 µm (Malvern Mastersizer Long bed ver. 2.19, focal length 45 mm, beam length 2.4 mm). Subsequently the emulsion was atomized in a spray tower, where the droplets were covered with starch and dried. Only part of the emulsion was sprayed. This yielded after screening on mesh 30/120 approx. 1 kg of particulate product with a potency of 252,000 IU/g (The sample was saponified with 50% KOH, 96% ethanol and a 10% sodium ascorbate solution and extracted with heptane. The amount of Vitamin A was measured by HPLC, Hichrom LiChrosorb CN-5, 5 µl, 250 mm x 4.0 mm against an external standard).

The stability of the product was investigated as follows: Approximately 0.2 gram product was weighed and placed in a small open glass container (15 x 10 mm) and kept at 25 °C/60% R.H. and at 40 °C/75% R.H. for 3 weeks. Samples were analyzed at start, after 7, 14 and 21 days for potency. The results obtained are given below:

| Potency: | 25°C/60% R.H. | 40°C/75% R.H. |
|---|---|---|
| Start: | 100% | 100% |
| 7 days: | 100% | 98% |
| 14 days: | 96% | 93% |
| 21 days: | 86% | 0.1 % |

As will appear from these results the potency and the stability of the composition of the invention were far superior to those of the prior art composition of the Comparative Example.

### Example 13

92.9 grams of rhamnogalacturonase modified sugar beet pectin prepared as described in example 4 and 797.0 grams of saccharose were dissolved in 1.0 I water at 65 °C in an emulsion tank and 12.6 grams of sodium ascorbate added. A mixture of 400.0 grams Vitamin A palmitate 1,7 million lU/g and 20.4 grams DL-α-tocopherol was heated to 65 °C in a beaker. The oily mixture was added to the aqueous solution of modified pectin, sugar and sodium ascorbate under slow agitation and then stirred vigorously at 10,000 rpm by Ultra Turrax (Ultra Turrax T50) for 60 minutes at 65 °C. The final emulsion was diluted with 980 ml 65 °C water to a viscosity of 150 cP (measured by Brookfield Viscometer type HAT, spindle HA1). The mean oil droplet size was measured to be 1.64 µm (Malvern Mastersizer Long bed ver. 2.19, focal length 45 mm, beam length 2.4 mm). Subsequently the emulsion was atomized in a spray tower, where the droplets were covered with starch and dried. Only part of the emulsion was sprayed. This yielded after screening on mesh 30/120 approx. 1 kg of particulate product with a potency of 218,000 IU/g (The sample was saponified with 50% KOH, 96% ethanol and a 10% sodium ascorbate solution and extracted with heptane. The amount of Vitamin A was measured by HPLC, Hichrom LiChrosorb CN-5, 5 µl, 250 mm x 4.0 mm against an external standard).

The stability of the product was investigated as follows: Approximately 0.2 gram product was weighed and placed in a small open glass container (15 x 10 mm) and kept at 25 °C/60% R.H. and at 40 °C/75% R.H. for 3 weeks. Samples were analyzed at start, after 7, 14 and 21 days for potency. The results obtained are given below:

| Potency: | 25°C/60% R.H. | 40°C/75% R.H. |
|---|---|---|
| Start: | 100% | 100% |
| 7 days: | 102% | 100% |
| 14 days: | 97% | 91 % |
| 21 days: | 91 % | 0.5% |

As will appear from these results the potency and the stability of the composition of the invention were far superior to those of the prior art composition of the Comparison Example.

### Example 14

92.9 grams of Pectinase PL lyase modified sugar beet pectin prepared as described in Example 5 and 797.0 grams of saccharose were dissolved in 1.0 l water at 65 °C in an emulsion tank and 12.6 grams of sodium ascorbate added. A mixture of 400.0 grams Vitamin A palmitate 1.7 million IU/g and 20.4 grams DL-α-tocopherol was heated to 65 °C in a beaker. The oily mixture was added to the aqueous solution of modified pectin, sugar and sodium ascorbate under slow agitation and then stirred vigorously at 10.000 rpm by Ultra Turrax (Ultra Turrax T50) for 60 minutes at 65°C. The final emulsion showed a viscosity of 155 cP (measured by Brookfield Viscometer type HAT, spindle HA1). The mean oil droplet size was measured to be 0.83 µm (Malvern Mastersizer Long bed ver. 2.19, focal length 45 mm, beam length 2.4 mm). Subsequently the emulsion was atomized in a spray tower, where the droplets were covered with starch and dried. Only part of the emulsion was sprayed. This yielded after screening on mesh 30/120 approx. 1 kg of particulate product with a potency of 349.000 IU/g (The sample was saponified with 50% KOH, 96% ethanol and a 10% sodium ascorbate solution and extracted with heptane. The amount of Vitamin A was measured by HPLC, Hichrom LiChrosorb CN-5, 5 µl, 250 mm x 4.0 mm against an external standard).

The stability of the product was investigated as follows: Approximately 0.2 gram product was weighed and placed in a small open glass container (15 x 10 mm) and kept at 25°C/60% R.H. and at 40°C/75% R.H. for 3 weeks. Samples were analyzed at start, after 7, 14 and 21 days for potency. The results obtained are given below:

| Potency: | 25°C/60% R.H. | 40°C/75% R.H. |
|---|---|---|
| Start: | 100% | 100% |
| 7 days: | 99% | 92% |
| 14 days: | 96% | 1.1% |
| 21 days: | 94% | 0.8% |

As will appear from these results the potency and the stability of the composition of the invention were far superior to those of the prior art composition of the Comparative Example.

### Example 15

92.9 grams of carboxypeptidase B modified sugar beet pectin prepared as described in Example 6 and 797.0 grams of saccharose were dissolved in 1.0 l water at 65 °C in an emulsion tank and 12.6 grams of sodium ascorbate added. A mixture of 400.0 grams Vitamin A palmitate 1.7 million IU/g and 20.4 grams DL-α-tocopherol was heated to 65 °C in a beaker. The oily mixture was added to the aqueous solution of modified pectin, sugar and sodium ascorbate under slow agitation and then stirred vigorously at 10.000 rpm by Ultra Turrax (Ultra Turrax T50) for 60 minutes at 65 °C. The final emulsion was diluted with 1151 ml 65 °C water to a viscosity of 155 cP (measured by Brookfield Viscometer type HAT, spindle HA1). The mean oil droplet size was measured to be 0.83 µm (Malvern Mastersizer Long bed ver. 2.19, focal length 45 mm, beam length 2.4 mm). Subsequently the emulsion was atomized in a spray tower, where the droplets were covered with starch and dried. Only part of the emulsion was sprayed. This yielded after screening on mesh 30/120 approx. 1 kg of particulate product with a potency of 222.000 IU/g (The sample was saponified with 50% KOH, 96% ethanol and a 10% sodium ascorbate solution and extracted with heptane. The amount of Vitamin A was measured by HPLC, Hichrom LiChrosorb CN-5, 5 µl, 250 mm x 4.0 mm against an external standard).

The stability of the product was investigated as follows: Approximately 0.2 gram product was weighed and placed in a small open glass container (15 x 10 mm) and kept at 25°C/60% R.H. and at 40°C/75% R.H. for 3 weeks. Samples were analyzed at start, after 7, 14 and 21 days for potency. The results obtained are given below:

| Potency: | 25°C/60% R.H. | 40°C/75% R.H. |
|---|---|---|
| Start: | 100% | 100% |
| 7 days: | 97% | 96% |
| 14 days: | 96% | 93% |
| 21 days: | 90% | 0.0% |

As will appear from these results the potency and the stability of the composition of the invention were far superior to those of the prior art composition of the Comparative Example.

### Example 16

92.9 grams of carboxypeptidase A modified sugar beet pectin prepared as described in Example 7 and 797.0 grams of saccharose were dissolved in 1.0 l water at 65 °C in an emulsion tank and 12.6 grams of sodium ascorbate added. A mixture of 400.0 grams Vitamin A palmitate 1.7 million lU/g and 20.4 grams DL-α-tocopherol was heated to 65 °C in a beaker. The oily mixture was added to the aqueous solution of modified pectin, sugar and sodium ascorbate under slow agitation and then stirred vigorously at 10.000 rpm by Ultra Turrax (Ultra Turrax T50) for 60 minutes at 65 °C. The final emulsion showed a viscosity of 100 cP (measured by Brookfield Viscometer type HAT, spindle HA1). The mean oil droplet size was measured to be 1.77 µm (Malvern Mastersizer Long bed ver. 2.19, focal length 45 mm, beam length 2.4 mm). Subsequently the emulsion was atomized in a spray tower, where the droplets were covered with starch and dried. Only part of the emulsion was sprayed. This yielded after screening on mesh 30/120 approx. 1 kg of particulate product with a potency of 331.000 lU/g (The sample was saponified with 50% KOH, 96% ethanol and a 10% sodium ascorbate solution and extracted with heptane. The amount of Vitamin A was measured by HPLC, Hichrom LiChrosorb CN-5, 5 µl, 250 mm x 4.0 mm against an external standard).

The stability of the product was investigated as follows: Approximately 0.2 gram product was weighed and placed in a small open glass container (15 x 10 mm) and kept at 25°C/60% R.H. and at 40°C/75% R.H. for 3 weeks. Samples were analyzed at start, after 7, 14 and 21 days for potency. The results obtained are given below:

| Potency: | 25°C/60% R.H. | 40°C/75% R.H. |
|---|---|---|
| Start: | 100% | 100% |
| 7 days: | 99% | 98% |
| 14 days: | 95% | 94% |
| 21 days: | 84% | 2.0% |

As will appear from these results the potency and the stability of the composition of the invention were far superior to those of the prior art composition of the Comparative Example.

### Example 17

92.9 grams of Flavorzyme modified sugar beet pectin prepared as described in Example 8 and 797.0 grams of saccharose were dissolved in 1.0 l water at 65 °C in an emulsion tank and 12.6 grams of sodium ascorbate added. A mixture of 400.0 grams Vitamin A palmitate 1.7 million IU/g and 20.4 grams DL-α-tocopherol was heated to 65 °C in a beaker. The oily mixture was added to the aqueous solution of modified pectin, sugar and sodium ascorbate under slow agitation and then stirred vigorously at 10.000 rpm by Ultra Turrax (Ultra Turrax T50) for 60 minutes at 65 °C. The final emulsion was diluted with 1093 ml 65 °C water to a viscosity of 155 cP (measured by Brookfield Viscometer type HAT, spindle HA1). The mean oil droplet size was measured to be 1.50 µm (Malvern Mastersizer Long bed ver. 2.19, focal length 45 mm, beam length 2.4 mm). Subsequently the emulsion was atomised in a spray tower, where the droplets were covered with starch and dried. Only part of the emulsion was sprayed. This yielded after screening on mesh 30/120 approx. 1 kg of particulate product with a potency of 203.000 IU/g (The sample was saponified with 50% KOH, 96% ethanol and a 10% sodium ascorbate solution and extracted with heptane. The amount of Vitamin A was measured by HPLC, Hichrom LiChrosorb CN-5, 5 µl, 250 mm x 4.0 mm against an external standard).

The stability of the product was investigated as follows: Approximately 0.2 gram product was weighed and placed in a small open glass container (15 x 10 mm) and kept at 25°C/60% R.H. and at 40°C/75% R.H. for 3 weeks. Samples were analyzed at start, after 7, 14 and 21 days for potency. The results obtained are given below:

| Potency: | 25°C/60% R.H. | 40°C/75% R.H. |
|---|---|---|
| Start: | 100% | 100% |
| 7 days: | 100% | 100% |
| 14 days: | 82% | 90% |
| 21 days: | 68% | 0.0% |

As will appear from these results the potency and the stability of the composition of the invention were far superior to those of the prior art composition of the Comparative Example.

### Example 18

92.9 grams of Pectinase PL lyase modified orange pectin prepared as described in Example 9 and 797.0 grams of saccharose were dissolved in 1.08 l water at 65 °C in an emulsion tank and 12.6 grams of sodium ascorbate added. A mixture of 400.0 grams Vitamin A palmitate 1.7 million IU/g and 20.4 grams DL-α-tocopherol was heated to 65 °C in a beaker. The oily mixture was added to the aqueous solution of modified pectin, sugar and sodium ascorbate under slow agitation and then stirred vigorously at 10.000 rpm by Ultra Turrax (Ultra Turrax T50) for 60 minutes at 65 °C. The final emulsion was diluted with 37 ml 65 °C water to a viscosity of 155 cP (measured by Brookfield Viscometer type HAT, spindle HA1). The mean oil droplet size was measured to be 1.84 µm (Malvern Mastersizer Long bed ver. 2.19, focal length 45 mm, beam length 2.4 mm). Subsequently the emulsion was atomised in a spray tower, where the droplets were covered with starch and dried. Only part of the emulsion was sprayed. This yielded after screening on mesh 30/120 approx. 1 kg of particulate product with a potency of 313.000 IU/g (The sample was saponified with 50% KOH, 96% ethanol and a 10% sodium ascorbate solution and extracted with heptane. The amount of Vitamin A is measured by HPLC, Hichrom LiChrosorb CN-5, 5 µl, 250 mm x 4.0 mm against an external standard).

The stability of the product was investigated as follows: Approximately 0.2 gram product was weighed and placed in a small open glass container (15 x 10 mm) and kept at 25°C/60% R.H. and at 40°C/75% R.H. for 3 weeks. Samples were analyzed at start, after 7, 14 and 21 days for potency. The results obtained are given below:

| Potency: | 25°C/60% R.H. | 40°C/75% R.H. |
|---|---|---|
| Start: | 100 % | 100 % |
| 7 days: | 99 % | 97 % |
| 14 days: | 98 % | 87 % |
| 21 days: | 93 % | 1 % |

As will appear from these results the potency and the stability of the composition of the invention were far superior to those of the prior art composition of the Comparative Example.

## Claims

1. Microcapsules comprising an active substance embedded in a matrix material, **characterized in that** the matrix material is obtainable by treating a pectic substance with one or more enzymes selected from the group consisting of esterases (E.C.3.1.), glucosidases (E.C.3.2.), peptidases (E.C.3.4.), proteases (E.C.3.4.) and lyases (E.C.4.).

2. Microcapsules according to claim 1, wherein the matrix material is obtainable by treating a pectic substance with one or more enzymes capable of modifying the hairy regions.

3. Microcapsules according to claims 1 or 2, wherein the matrix material is obtainable by treating a pectic substance with one or more enzymes capable of modifying the backbone of the smooth regions so as to form separate elements comprising hairy regions.

4. Microcapsules according to claim 1, wherein the matrix material is obtainable by treating a pectic substance with papain, pepsin or trypsin.

5. Microcapsules according to claim 4, wherein the matrix material is obtainable by treating the pectic substance with papain, such as Collupulin©.

6. Microcapsules according to claim 1, wherein the matrix material is obtainable by treating the pectic substance with an exopeptidase, such as carboxypeptidase.

7. Microcapsules according to claims 1-3, wherein the matrix material is obtainable by treating a pectic substance with a deacetylating enzyme.

8. Microcapsules according to claims 1-3, wherein the matrix material is obtainable by treating a pectic substance with a debranching enzyme.

9. Microcapsules according to claims 1-3, wherein the matrix material is obtainable by treating a pectic substance with a mixture of α-arabinofuranosidase, galactanase, and arabinanase.

10. Microcapsules according to claim 1-3, wherein the matrix material is obtainable by treating the pectic substance with a lyase, such as pectinase PL lyase.

11. Microcapsules according to any of claims 1-10, wherein the pectic substance is derived from a vegetable.

12. Microcapsule according to any of claims 1-10, wherein the pectic substance is derived from orange, grape, sugar beet, soybean, linseed, Jerusalem artichoke, celery, potatoes and beetroots.

13. A process of preparing microcapsules containing an active substance embedded in a matrix material comprising the steps of providing an aqueous medium of a pectic substance modified by treatment with one or more enzymes selected from the group consisting of esterases (E.C.3.1), glucosidases (E.C.3.2), peptidases (E.C.3.4), proteases (E.C.3.4), and lyases (E.C.4), adding to said solution at least one active substance, finely dividing and drying the mixture thus obtained to obtain a mass of particles each containing a plurality of liquid or solid micro particles of the active substance embedded in a matrix comprising the modified pectic substance.

14. A product comprising microcapsules according to any of claims 1-12.

15. A product according to claim 14, **characterized in that** it is a food, a food supplement, a beverage, a pharmaceutical or veterinary product, a feed or feed supplement, a personal care product or a household product.

## Patentansprüche

1. Mikrokapseln, umfassend eine Wirksubstanz, eingebettet in ein Matrixmaterial, **dadurch gekennzeichnet, dass** das Matrixmaterial durch Behandeln einer Pektinsubstanz mit ein oder mehreren Enzymen, ausgewählt aus der Gruppe, bestehend aus Esterasen (E.C.3.1.), Glucosidasen (E.C.3.2.), Peptidasen (E.C.3.4.), Proteasen (E.C.3.4.) und Lyasen (E.C.4.), erhaltbar ist.

2. Mikrokapseln nach Anspruch 1, worin das Matrixmaterial durch Behandeln einer Pektinsubstanz mit ein oder mehreren Enzymen, die zum Modifizieren der neutralzuckerreichen Zonen (hairy regions) fähig sind, erhaltbar ist.

3. Mikrokapseln nach Anspruch 1 oder 2, worin das Matrixmaterial durch Behandeln einer Pektinsubstanz mit ein oder mehreren Enzymen erhaltbar ist, die zum Modifizieren des Rückgrats der homogenen Polygalacturonsäure-Abschnitte (smooth regions) fähig sind, um dabei separate Elemente, umfassend hairy regions, zu bilden.

4. Mikrokapseln nach Anspruch 1, worin das Matrixmaterial durch Behandeln einer Pektinsubstanz mit Papain, Pepsin oder Trypsin erhaltbar ist.

5. Mikrokapseln nach Anspruch 4, worin das Matrixmaterial durch Behandeln der Pektinsubstanz mit Papain, wie etwa Collupulin©, erhaltbar ist.

6. Mikrokapseln nach Anspruch 1, worin das Matrixmaterial durch Behandeln der Pektinsubstanz mit einer Exopeptidase, wie etwa Carboxypeptidase, erhaltbar ist.

7. Mikrokapseln nach Anspruch 1-3, worin das Matrixmaterial durch Behandeln einer Pektinsubstanz mit einem deacetylierenden Enzym erhaltbar ist.

8. Mikrokapseln nach Anspruch 1-3, worin das Matrixmaterial durch Behandeln einer Pektinsubstanz mit einem entzweigenden Enzym erhaltbar ist.

9. Mikrokapseln nach Anspruch 1-3, worin das Matrixmaterial durch Behandeln einer Pektinsubstanz mit einem Gemisch aus α-Arabinofuranosidase, Galactanase und Arabinanase erhaltbar ist.

10. Mikrokapseln nach Anspruch 1-3, worin das Matrixmaterial durch Behandeln der Pektinsubstanz mit einer Lyase, wie etwa Pectinase PL-Lyase, erhaltbar ist.

11. Mikrokapseln nach einem der Ansprüche 1-10, worin die Pektinsubstanz von einem Gemüse stammt.

12. Mikrökapseln nach einem der Ansprüche 1-10, worin die Pektinsubstanz von Orange, Traube, Zuckerrübe, Sojabohne, Leinsamen, Jerusalem-Artischocke, Selerie, Kartoffeln und Rote Bete stammt.

13. Verfahren zum Herstellen von Mikrokapseln, die eine Wirksubstanz, eingebettet in ein Matrixmaterial, enthalten, umfassend die Schritte des Bereitstellens eines wässrigen Mediums von einer Pektinsubstanz, modifiziert durch Behandeln mit ein oder mehreren Enzymen, ausgewählt aus der Gruppe, bestehend aus Esterasen (E.C.3.1.), Glucosidasen (E.C.3.2.), Peptidasen (E.C.3.4.), Proteasen (E.C.3.4.) und Lyasen (E.C.4.); Zugeben zu der Lösung wenigstens einer Wirksubstanz, Feinzerteilen und Trocknen des derart erhaltenen Gemischs, um eine Masse von Partikeln zu erhalten, die jeweils eine Vielzahl von flüssigen oder festen Mikropartikeln der Wirksubstanz, eingebettet in eine Matrix, umfassend die Modifizierte Pektinsubstanz, enthalten.

14. Produkt, umfassend Mikrokapseln nach einem der Ansprüche 1-12.

15. Produkt nach Anspruch 14, **dadurch gekennzeichnet, dass** es ein Nahrungsmittel, eine Nahrungsmittelergänzung, ein Getränk, ein pharmazeutisches oder veterinärpharmazeutisches Produkt, ein Futtermittel oder eine Futtermittelergänzung, ein persönliches Pflegeprodukt oder ein Haushaltsprodukt ist.

## Revendications

1. Microcapsules comprenant une substance active enrobée dans une matière de matrice, **caractérisées en ce que** la matière de matrice est obtenue en traitant une substance pectique avec une ou plusieurs enzymes sélectionnées parmi le groupe contenant des estérases (E.C.3.1), des glucosidases (E.C.3.2), des peptidases (E.C.3.4), des protéases (E.C.3.4) et des lyases (E.C.4.).

2. Microcapsules selon la revendication 1, dans lesquelles la matière de matrice est obtenue en traitant une substance pectique avec une ou plusieurs enzymes capables de modifier les régions ramifiées.

3. Microcapsules selon les revendications 1 ou 2, dans lesquelles la matière de matrice est obtenue en traitant une substance pectique avec une ou plusieurs enzymes capables de modifier le squelette des régions lisses afin de former des éléments séparés comprenant des régions ramifiées.

4. Microcapsules selon la revendication 1, dans lesquelles la matière de matrice est obtenue en traitant une substance pectique avec de la papaïne, de la pepsine ou de la trypsine.

5. Microcapsules selon la revendication 4, dans lesquelles la matière de matrice est obtenue en traitant une substance pectique avec de la papaïne, telle que du Collupulin©.

6. Microcapsules selon la revendication 1, dans lesquelles la matière de matrice est obtenue en traitant une substance pectique avec une exopeptidase, telle que la carboxypeptidase.

7. Microcapsules selon les revendications 1 à 3, dans lesquelles la matière de matrice est obtenue en traitant une substance pectique avec une enzyme de déacétylation.

8. Microcapsules selon les revendications 1 à 3, dans lesquelles la matière de matrice est obtenue en traitant une substance pectique avec une enzyme de débranchement.

9. Microcapsules selon les revendications 1 à 3, dans lesquelles la matière de matrice est obtenue en traitant une substance pectique avec un mélange d'α-arabinofuranosidase, de galactanase, et d'arabinanase.

10. Microcapsules selon les revendications 1 à 3, dans lesquelles la matrice est obtenue en traitant une substance pectique avec une lyase, telle que la pectine lyase PL.

11. Microcapsules selon l'une des revendications 1 à 10, dans lesquelles la substance pectique est dérivée d'un végétal.

12. Microcapsules selon l'une des revendications 1 à 10, dans lesquelles la substance pectique est dérivée d'orange, de raisin, de sucre de betterave, de graine de soja, de graine de lin, d'artichaut de Jérusalem, de céleri, de pommes de terre et de betteraves.

13. Procédé de préparation de microcapsules contenant une substance active enrobée dans une matière de matrice comprenant les étapes de préparation d'un milieu aqueux d'une substance pectique modifiée par traitement avec une ou plusieurs enzymes sélectionnées parmi le groupe contenant des estérases (E.C.3.1), des glucosidases (E.C.3.2), des peptidases (E.C.3.4), des protéases (E.C.3.4) et des lyases (E.C.4.), d'addition de ladite solution à au moins une substance active, de division fine et de séchage du mélange obtenu, pour obtenir une masse de particules chacune contenant une pluralité de microparticules liquides ou solides de substance active enrobée dans une matière de matrice comprenant la substance pectique modifiée.

14. Produit comprenant des microcapsules selon l'un des revendications 1 à 12.

15. Produit selon la revendication 14, **caractérisé en ce qu'**il est un aliment, un supplément alimentaire, une boisson, un produit pharmaceutique ou vétérinaire, de la nourriture ou un supplément de nourriture, un produit de soins personnel ou un produit d'entretien domestique.
